# EUROPEAN PATENT SPECIFICATION

(11) **EP 3 217 971 B1**
(45) Date of publication and mention of the grant of the patent: **11.09.2019**
(21) Application number: 15805005.4
(22) Date of filing: 10.11.2015
(51) Int. Cl.: A61K 31/351, A61P 1/12, A61K 31/35, A23K 20/195, A23K 50/30, A23K 50/60

(54) **ANTIVIRAL EFFECTS OF NARASIN IN SWINE FEED**
ANTIVIRALE WIRKUNGEN VON NARASIN IN FUTTERMITTEL FÜR SCHWEINE
EFFETS ANTIVIRAUX DU NARASIN DANS DE LA NOURRITURE POUR PORCS

(30) Priority: 13.11.2014 US 201462079159 P
(43) Date of publication of application: 20.09.2017
(73) Proprietor: Elanco US Inc., Greenfield, IN 46140 (US)
(72) Inventor: MARSTELLER, Thomas Alan, Indianapolis, Indiana 46206-6288 (US); OWENS, Jane Granville, Indianapolis, Indiana 46206-6288 (US); PULS, Christopher Leigh, Indianapolis, Indiana 46206-6288 (US); RITTER, Matthew John, Indianapolis, Indiana 46206-6288 (US); ROSENKRANS, Kelly Shern, Indianapolis, Indiana 46206-6288 (US); WEBER, Thomas Edmund, Indianapolis, Indiana 46206-6288 (US)
(74) Representative: Bassinder, Emma Marie
(86) International application number: PCT/US2015/059848
(87) International publication number: WO 2016/077282

(56) References cited:
- WO-A1-2014/121342
- KR-A- 20110 039 079
- US-A- 4 174 404
- US-A- 5 834 473

## Description

The present invention relates to compositions containing narasin and methods of using narasin to treat a nursery pig for porcine epidemic diarrhea virus infection.

The present invention is in the field of treatment of symptoms associated with porcine epidemic diarrhea virus (PEDV) infection. PEDV is a coronavirus which causes mortality in up to 100% of infected piglets, but has limited mortality in older swine. More than 10,000 piglets may die each day in the United States from PEDV. Thus, PEDV is having a significant economic impact on the price and availability of pork products.

Two PEDV vaccines are currently being marketed in the United States. Both vaccines have been approved for use in pregnant sows which may provide passive immunity to nursing piglets through antibodies in the sows' milk. However, the efficacy of these vaccines has yet to be demonstrated, the vaccines must be administered to sows individually through injection, and the levels of maternally-derived antibodies begin to decline after closure of a piglet's gut to macromolecule absorption. WO2014/121342 discloses the use of *Epimedium koreanum* extract for preventing and treating porcine epidemic diarrhea.

Narasin is a polyether ionophore produced by *Streptomyces spp,* and can be purified from cultures of *S. lydicus* and *S. granuloruber.* Narasin has been approved by regulatory authorities in many countries to increase weight gain in growing-finishing swine. Narasin has been shown to block replication of the flavivirus responsible for dengue fever when added to cultured human cells infected with the virus (Low, et al., Antiviral Therapy 16: 1203-18, 2011). However, when administered with feed, narasin has been reported to be toxic to nursery pigs when supplied at a concentration of about 83 mg/kg feed, at least in the presence of tiamulin. The use of deoxynarasin antibiotics for treating swine dysentery is disclosed in KR20110039079. The use of compositions comprising polyether ionophore such as narasin for treating microbial infections in animals including swine is disclosed in US4174404. The use of narasin for treating coccidiosis is also known from US5834473.

New treatments for PEDV are needed, especially a therapy which would provide protection to a pigs after weaning, such as a nursery pig. A therapy which can be administered orally to a number of animals at once would also be advantageous. Because PEDV has been shown to be transmitted by aerosolized virus, a therapy which would decrease shedding of virus from infected swine would facilitate containment of the disease caused by this virus.

Accordingly, the present invention provides narasin for use in the treatment of PEDV infection in a nursery pig. Narasin may be supplied to the nursery pig as a composition with an orally-acceptable carrier such as, for example without limitation, an animal feed, a liquid composition other than an animal feed, or a solid composition other than an animal feed. The concentration of narasin present in this composition may be about 30 mg/kg, about 40 mg/kg, about 50 mg/kg, or about 60 mg/kg of the orally-acceptable carrier. The present invention also provides narasin for use in the treatment of PEDV infection in a nursery pig, wherein narasin is administered with an animal feed and the concentration of narasin is about 30 mg/kg to about 60 mg/kg of the animal feed. The present invention also provides narasin for use in the treatment of PEDV infection in a nursery pig, wherein narasin is administered with an animal feed and the concentration of narasin is about 60 mg/kg of the animal feed.

Further, the present invention provides narasin with an orally-acceptable carrier for use in the treatment of PEDV infection in a nursery pig, wherein the orally-acceptable carrier comprises an animal feed, a liquid composition other than an animal feed, or a solid composition other than an animal feed, and wherein the concentration of narasin may be about 30 mg/kg, about 40 mg/kg, about 50 mg/kg, or about 60 mg/kg of the orally-acceptable carrier. Further, the present invention also provides narasin with an animal feed for use in the treatment of PEDV infection in a nursery pig, wherein the concentration of narasin is about 30 mg/kg to about 60 mg/kg of the animal feed. Further, the present invention also provides narasin with an animal feed for use in the treatment of PEDV infection in a nursery pig, wherein the concentration of narasin is about 60 mg/kg of the animal feed.

Also disclosed is the use of narasin in the preparation of a medicament for the treatment of PEDV infection in a nursery pig. The medicament may be a composition comprising narasin with an orally-acceptable carrier such as, for example without limitation, an animal feed, a liquid composition other than an animal feed, or a solid composition other than an animal feed. The concentration of narasin present in this composition may be about 30 mg/kg, about 40 mg/kg, about 50 mg/kg, or about 60 mg/kg of the orally-acceptable carrier. Further disclosed herein is the use of narasin in the preparation of a medicament for the treatment of PEDV infection in a nursery pig, wherein the medicament is narasin with an animal feed and the concentration of narasin is about 30 mg/kg to about 60 mg/kg of the animal feed. Further disclosed herein is the use of narasin in the preparation of a medicament for the treatment of PEDV infection in a nursery pig, wherein the medicament is narasin with an animal feed and the concentration of narasin is about 60 mg/kg of the animal feed.

Also disclosed is a composition which provides an antiviral effect to a nursery pig, wherein the composition comprises a concentration of narasin and an orally-acceptable carrier. The orally-acceptable carrier may comprise an animal feed, a liquid composition other than an animal feed, or a solid composition other than an animal feed. The concentration of narasin in the above composition may be about 30 mg/kg, about 40 mg/kg, about 50 mg/kg, or about 60 mg/kg of the composition. Further disclosed is a composition which provides an antiviral effect to a nursery pig, wherein the composition comprises a concentration of narasin with an animal feed, wherein the concentration of narasin is about 30 mg/kg to about 60 mg/kg of the animal feed. Further disclosed is a composition which provides an antiviral effect to a nursery pig, wherein the composition comprises a concentration of narasin with an animal feed, wherein the concentration of narasin is about 60 mg/kg of the animal feed.

Further disclosed is a composition comprising a concentration of narasin and an orally-acceptable carrier, said composition providing an antiviral effect to a nursery pig, wherein the orally-acceptable carrier is selected from the group of an animal feed, a liquid composition other than an animal feed, and a solid composition other than an animal feed, and wherein the concentration of narasin may be about 30 mg/kg, about 40 mg/kg, about 50 mg/kg, and about 60 mg/kg of the orally-acceptable carrier.

Narasin and methods of making and using narasin as a useful therapeutic against gram-positive bacteria, anaerobic bacteria, and fungi, as an anticocccidial agent, and as an agent for increasing feed utilization in ruminants, are recited in U.S. Pat No. 4,038,384 (published July 26, 1977), U.S. Pat. No. 4,309,504 (published Jan. 5, 1982), and U.S. Pat. No. 4,342,829 (published Aug. 3, 1982). See also Berg et al., J. Antibiot. 31: 1-6 (1978) and "Narasin, a new polyether antibiotic: discovery and fermentation studies, Chapter 38, pages 471-485, volume 18, Developments in Industrial Microbiology [a publication of the Society for Microbiology (1977)].

As used herein, the terms "treating", "to treat", or "treatment", include restraining, slowing, stopping, reducing, ameliorating, or reversing the progression or severity of an existing symptom, disorder, condition, or disease. A treatment may be applied prophylactically or therapeutically.

As used herein, "nursery pig" is a pig which has been weaned but is not yet a growing-finishing pig. Weaning of pigs typically occurs at about three weeks of age (about 21 days old) but can occur as early as one week (about seven days old) and as late as six weeks of age (about 42 days old). A weaned pig no longer solely relies on a sow's milk for sustenance but rather consumes solid feed compositions.

As used herein, "growing-finishing swine" are pigs of at least about 50 pounds of body weight, or about ten weeks of age. The terms "growing-finishing," grow-finish," and "grower-finisher" are synonymous. The term "swine" includes any member of the genus *Sus.*

As used herein, "animal feed" includes edible materials which are consumed by livestock for the materials' nutritional value. Animal feed includes feed rations, e.g. compositions that meet an animal's nutritional requirements, and also include compositions that do not meet an animal's nutritional requirement.

In an embodiment, the composition comprises an orally-acceptable carrier for narasin. An "orally-acceptable carrier" includes any physiologically acceptable carrier suitable for oral administration. Orally-acceptable carriers include, without limitation, animal feed compositions, aqueous compositions, and liquid and solid compositions suitable for use in animal feed products and/or for oral administration to an animal. Suitable carriers are known in the art, and include those described in U.S. Patent 6,780,628.

The following experimental example is illustrative of the use of narasin to reduce viral shedding or ameliorate symptoms of viral infection in nursery pigs. The invention is not limited to this specific illustrative example or to any preferred embodiment, and the invention could apply to treatments for other viruses, such as porcine reproductive and respiratory syndrome (PRRS) virus, porcine circovirus (PCV), or a porcine coronavirus.

### Example 1

### Experimental Design

Eighty-one weaning stage (21.8 +/- 0.6 days) commercial crossbred piglets (Wilson's Prairie View Farms, Burlington, WI) free of PEDV infection arrived at the site and six were used as potential replacement animals during the pre-challenge period (Day -7 to Day 0). Six piglets were removed at Day 0 immediately prior to PEDV challenge, resulting in fifteen pens containing five piglets/pen (n = 75) animals. There were five pens per treatment. For balance of sentinel piglets within a block, a total of thirty-nine piglets of one gender and thirty-six of the other gender were used. The use of five pens (twenty-five piglets) per treatment group was estimated to detect significant differences (*P* < 0.10) between treatment groups in growth performance and incidence of viral shedding.

The seventy-five piglets were randomly assigned to one of three treatment groups: control (no ionophore), narasin (30mg/kg) and narasin (60mg/kg). They acclimatized to their environment and were fed daily for seven days prior to exposure with PEDV on Day 0. Treatment continued for 14 days following challenge. Pigs from the three treatment groups were administered orally with 4x10⁴ TCID₅₀/mL of the PEDV isolate PEDV/USA/NC/2013/49469 (College of Veterinary Medicine, Iowa State University). The piglets were monitored daily for changes in clinical parameters, , clinical score for diarrhea, depression and gauntness, rate of food consumption, and rate of body weight change and viral swabs were taken daily to determine viral shedding.

### Statistical Methods

Viral shedding values was evaluated by RMANOVA and the PROC MIXED procedure of SAS (SAS, Cary, NC). Other variables for analysis included growth performance data (average daily gain, or ADG; average daily feed intake, or ADFI; and feed efficiency (unit of weight gain per unit of feed consumed), or G:F), incidence and severity of diarrhea, depression, and gauntness scores, and intestinal histology score and immunohistochemistry. The analysis of the growth performance outcomes were conducted using a generalized linear mixed model and the PROC MIXED procedure of SAS.

Incidence and severity of the score data (diarrhea, depression, and gauntness) were summarized by frequencies and mean scores on a daily basis.

### Diet Formulations

Diet formulations were manufactured at Provimi (Lewisburg, OH) and fed in meal form. Composition of diets were analyzed by Minnesota Valley Testing Laboratories (New Ulm, MN), and nutrient analysis values were found to be similar to formulated levels. Narasin levels were analyzed by Covance Laboratories (Greenfield, IN) and were found to be 0, 29.8, and 64.1 mg/kg, which were similar to formulated levels of 0, 30, and 60 mg/kg, respectively.

No ractopamine (last feed drug) was detected in the experimental diets.

### Growth Performance

The effects of narasin inclusion level on mean body weights (BW), average daily weight gain (ADG), average daily feed intake (ADFI), feed efficiency (G:F), and feed conversion ratio (F:G) are shown in Table 1.

**Table 1. Least-squares means for the effects of narasin inclusion level on the growth performance of nursery pigs challenged with PEDV**

| Narasin inclusion level, mg/kg | | | | | |
|---|---|---|---|---|---|
| Item | 0 | 30 | 60 | SEM | *P*-value |
| No. of pens | 5 | 5 | 5 | - | - |

| BW, lb | | | | | |
|---|---|---|---|---|---|
| Day -7¹ | 14.3 | 14.9^{*} | 14.9* | 0.72 | 0.04 |
| Day 0² | 16.6 | 16.9 | 16.7 | 0.68 | 0.72 |
| Day 5³ | 18.1 | 18.7 | 19.2 | 0.78 | 0.12 |
| Day 14⁴ | 26.5 | 26.9 | 26.9 | 1.01 | 0.92 |

| ADG, lb | | | | | |
|---|---|---|---|---|---|
| Day -7 to 0 | 0.32 | 0.29 | 0.26* | 0.017 | 0.08 |
| Day 0 to 5 | 0.24 | 0.38* | 0.48* | 0.056 | 0.03 |
| Day 5 to 14 | 0.94 | 0.92 | 0.85 | 0.066 | 0.61 |
| Day 0 to 14 | 0.65 | 0.70 | 0.70 | 0.047 | 0.74 |

| ADFI, lb | | | | | |
|---|---|---|---|---|---|
| Day -7 to 0 | 0.36 | 0.37 | 0.36 | 0.032 | 0.67 |
| Day 0 to 5 | 0.65 | 0.68 | 0.71 | 0.035 | 0.51 |
| Day 5 to 14 | 1.23 | 1.23 | 1.22 | 0.102 | 1.00 |
| Day 0 to 14 | 0.99 | 1.00 | 1.01 | 0.069 | 0.98 |

| G:F, lb:lb | | | | | |
|---|---|---|---|---|---|
| Day -7 to 0 | 0.882 | 0.802 | 0.742 | 0.0755 | 0.40 |
| Day 0 to 5 | 0.375 | 0.551 | 0.670* | 0.0767 | 0.05 |
| Day 5 to 14 | 0.772 | 0.772 | 0.708 | 0.0609 | 0.59 |
| Day 0 to 14 | 0.659 | 0.711 | 0.698 | 0.0486 | 0.73 |

| F:G, lb:lb | | | | | |
|---|---|---|---|---|---|
| Day -7 to 0 | 1.14 | 1.33 | 1.38 | 0.112 | 0.22 |
| Day 0 to 5 | 3.80 | 1.93 | 1.56 | 0.744 | 0.12 |
| Day 5 to 14 | 1.30 | 1.38 | 1.42 | 0.114 | 0.64 |
| Day 0 to 14 | 1.52 | 1.48 | 1.44 | 0.106 | 0.85 |

| | | | | | |
|---|---|---|---|---|---|
| ^{*}Means are significantly different than control treatment (*P* < 0.10). ¹Day -7: Animal arrival to test facility; allotment to study. Includes extra 6 pigs. ²Day 0: Pigs challenged with PEDV. ³Day 5: Sentinel animal (1 pig/pen) removed from pen, euthanized, and necropsied. ⁴Day 14: End of study. | | | | | |

Feeding 30 and 60 mg/kg narasin numerically increased overall ADG (7.7% and 7.7%, respectively) and G:F (7.9% and 5.9%, respectively) compared to the control (0 mg/kg).

Feeding 30 and 60 mg/kg narasin increased (*P* < 0.10) ADG (58.3% and 100%, respectively) in the Day 0 to 5 period (Table 1) compared to the control, although feeding 60 mg/kg narasin resulted in a decreased (*P* < 0.10) ADG (18.8%) in the Day -7 to 0 period. In addition, feeding 60, but not 30 mg/kg narasin, increased (*P* < 0.10) G:F in the Day 0 to 5 period (78.7%) compared to 0 mg/kg narasin. There were no statistical differences for ADG, ADFI, G:F, or F:G in the Day 5 to 14 period. There was no effect (*P* > 0.10) of narasin level on overall (Day 0 to 14) growth performance.

### Clinical Scoring

Table 2 indicates the scoring system used to measure diarrhea, depression, and gauntness. Table 3 compares the mean scores for diarrhea, depression and gauntness in nursery pigs whose diet included 0, 30 or 60 mg/kg narasin.

**Table 2. Enteric Clinical Observation Scoring System**

| **Clinical Evaluation** | **Clinical Signs** |
|---|---|
| Diarrhea | 1 = Normal (no diarrhea present) |
| | 2 = Pasty (semi-solid; cow-pie consistency) |
| | 3 = Semi-liquid (loose with some solid material; oatmeal consistency) |
| | 4 = Liquid (watery feces with little or no solid material) |
| Depression | 0 = Normal (bright, alert, and responsive) |
| | 1 = Mild (may stand isolated but will quickly respond to stimulation) |
| | 2 = Moderate (may stand isolated with head down and possible signs of muscle weakness; delayed response to stimulation) |
| | 3 = Severe (severely depressed; recumbent and reluctant to rise) |
| Gauntness | 0 = Normal abdominal fill; flank is full and round |
| | 1 = Decreased gut fill; flank is flat |
| | 2 = Severely gaunt; flank is hollow |

The mean of diarrhea scores measured over the 14-d period decreased numerically with increasing narasin level (1.49, 1.45, and 1.37 for 0, 30, and 60 mg/kg, respectively). Severity of diarrhea decreased with increasing narasin level (3.16, 2.72, and 2.48 for 0, 30, and 60 mg/kg, respectively; Table 3).

The mean of depression scores measured over the 14-d period was not affected by narasin level, however, a low incidence of depression observed in animals is noted (5, 4, and 3 animals for 0, 30, and 60 mg/kg narasin, respectively). Severity of depression decreased with increasing narasin level, although this was based on relatively few animals exhibiting the condition (Table 3).

There was little effect of narasin level on mean or severity of gauntness scores measured over the 14-d period (Table 3).

**Table 3. Means for the effects of narasin inclusion level on diarrhea scores of nursery pigs challenged with PEDV**

| | Narasin inclusion level, mg/kg | | |
|---|---|---|---|
| Item | 0 | 30 | 60 |
| No. of pens | 5 | 5 | 5 |

| Diarrhea score | | | |
|---|---|---|---|
| Mean of Day 0 to 14 | 1.49 | 1.45 | 1.37 |
| Severity¹ | 3.16 | 2.72 | 2.48 |
| Incidence | 22/25 | 19/25 | 19/25 |

| Depression score | | | |
|---|---|---|---|
| Mean of Day 0 to 14 | 0.02 | 0.03 | 0.02 |
| Severity¹ | 0.20 | 0.16 | 0.12 |
| Incidence | 5/25 | 4/25 | 3/25 |

| Gauntness score¹ | | | |
|---|---|---|---|
| Mean of Day 0 to 14 | 0.19 | 0.14 | 0.14 |
| Severity¹ | 0.64 | 0.68 | 0.52 |
| Incidence | 14/25 | 14/25 | 11/25 |

| | | | |
|---|---|---|---|
| ¹Severity: Average of worst score observed for each pig over 14-d period. | | | |

### Viral shedding

The effects of narasin levels (0, 30, 60 mg/kg) on within-day viral shedding of nursery pigs (Table 4) and percentage of nursery pigs shedding PEDV (Table 5) are compared. Fecal swabs were collected from all pigs on test daily from Day 0 to 14 of study. Each sample was labeled with pig identification, pen number, and sample day. All samples were frozen at the time of collection and stored until required for analysis. Only fecal swab samples collected from days 0, 1, 2, 3, 4, 5, 6, 7, 8, 9, and 14 were sent for analysis. Fecal swab samples were sent to the Iowa State Veterinary Diagnostic Laboratory (ISVDL) and were analyzed using real-time RT-PCR to determine viral shedding of PEDV.

**Table 4. Least-squares means for the effects of narasin inclusion level within day on viral shedding of nursery pigs challenged with PEDV**

| | *P*-values | | |
|---|---|---|---|
| Item | Narasin | Day | Narasin × Day |
| Viral shedding | 0.01 | <0.0001 | <0.0001 |
| | Narasin inclusion level, mg/kg | | |
| Item | 0 | 30 | 60 |
| No. of pens | 5 | 5 | 5 |

| Viral shedding, cells/mL^{1,2} | | | |
|---|---|---|---|
| Day 0 | 0 | 0 | 0 |
| Day 1 | 1 | 1 | 0 |
| Day 2 | 802,918 | 1,005* | 1* |
| Day 3 | 46,321,178 | 152,889* | 4,085* |
| Day 4 | 299,600,217 | 7,543,717 | 18,980* |
| Day 5 | 2,577,353,893 | 108,303,640 | 593,842* |
| Day 6 | 2,546,739,463 | 879,972,048 | 5,551,647* |
| Day 7 | 2,642,072,514 | 1,422,120,182 | 171,586,154 |
| Day 8 | 83,710,799 | 268,967,479 | 58,813,444 |
| Day 9 | 5,615,075 | 63,817,042 | 16,189,481 |
| Day 14 | 3,665 | 2,256 | 9,262 |

| | | | |
|---|---|---|---|
| ^{*}Within day, means are significantly different than control treatment (*P* < 0.10). ¹Values were ln(count + 1) transformed prior to the statistical analysis. ²Values presented are only from pigs testing positive for shedding. Values for pigs testing negative (i.e., 0) are not included in the data set. | | | |

When compared to the control, pigs fed 30 mg/kg narasin had lower (P < 0.10) viral shedding on Day 2 and Day 3 of study, and consistently had numerically lower viral shedding through Day 7 of study. Pigs fed 60 mg/kg narasin had lower (P < 0.10) viral shedding on Day 2, 3, 4, 5, and 6 of study, and had numerically lower viral shedding on Day 7 and 8 of study (Table 4).

There was no effect (P > 0.10) of narasin level on the percentage of pigs shedding PEDV, but the duration of shedding generally decreased as narasin level increased.

### Histology

The effects of narasin inclusion level on the intestinal histology score and immunohistochemistry were analyzed. As narasin level increased from 0, 30, 60 mg/kg), histology scores decreased from 2 to 0 while the immunohistochemistry score decreased only at the 60mg/kg level (from 3 to 2) although differences were not significant (*P* > 0.05).

## Claims

1. Narasin for use in the treatment of porcine epidemic diarrhea virus (PEDV) infection in a nursery pig.

2. Narasin for use according to claim 1, wherein narasin is in a composition with an orally-acceptable carrier selected from the group comprising an animal feed, a liquid composition other than an animal feed, and a solid composition other than an animal feed.

3. Narasin for use according to claim 2, wherein narasin is present in the composition at a concentration of about 30 mg/kg, about 40 mg/kg, about 50 mg/kg, or about 60 mg/kg of the orally-acceptable carrier.

## Patentansprüche

1. Narasin zur Verwendung in der Behandlung einer Infektion durch Porziner Epidemischer Virusdiarrhoe (PEDV) bei einem Babyschwein.

2. Narasin zur Verwendung nach Anspruch 1, wobei Narasin in einer Zusammensetzung mit einem oral verträglichen Träger vorliegt, der aus der Gruppe ausgewählt ist, die ein Tierfutter, eine andere flüssige Zusammensetzung als ein Tierfutter und eine andere feste Zusammensetzung als ein Tierfutter umfasst.

3. Narasin zur Verwendung nach Anspruch 2, wobei Narasin in der Zusammensetzung in einer Konzentration von etwa 30 mg/kg, etwa 40 mg/kg, etwa 50 mg/kg oder etwa 60 mg/kg des oral verträglichen Trägers vorliegt.

## Revendications

1. Narasine pour une utilisation dans le traitement d'une infection par le virus de la diarrhée épidémique porcine (PEDV) chez un porc de pouponnière.

2. Narasine pour une utilisation selon la revendication 1, dans laquelle la narasine est dans une composition avec un véhicule oralement acceptable choisi dans le groupe comprenant un aliment animal, une composition liquide différente d'un aliment animal et une composition solide différente d'un aliment animal.

3. Narasine pour une utilisation selon la revendication 2, dans laquelle la narasine est présente dans la composition à une concentration d'environ 30 mg/kg, d'environ 40 mg/kg, d'environ 50 mg/kg ou d'environ 60 mg/kg du véhicule oralement acceptable.
